# EUROPÄISCHE PATENTSCHRIFT

(11) **EP 1 562 565 B1**
(45) Veröffentlichungstag und Bekanntmachung des Hinweises auf die Patenterteilung: **12.03.2008**
(21) Anmeldenummer: 03779881.6
(22) Anmeldetag: 10.11.2003
(51) Int. Cl.: A61K 31/02, A61K 33/24

(54) **VERWENDUNG EINES ODER MEHRERER DER ELEMENTE AUS DER GRUPPE YTTRIUM, NEODYM UND ZIRCONIUM**
USE OF ONE OR MORE ELEMENTS FROM THE GROUP CONTAINING YTTRIUM, NEODYMIUM AND ZIRCONIUM
UTILISATION D'UN OU DE PLUSIEURS ELEMENTS DU GROUPE RASSEMBLANT L'YTTRIUM, LE NEODYME ET LE ZIRCONIUM

(30) Priorität: 13.11.2002 DE 10253634
(43) Veröffentlichungstag der Anmeldung: 17.08.2005
(73) Patentinhaber: Biotronik GmbH & Co. KG, 12359 Berlin (DE)
(72) Erfinder: HARDER, Claus Dr., 91080 Uttenreuth (DE); HEUBLEIN, Bernd Prof. Dr., verstorben (DE); HEUBLEIN, Eva Dr. med., 30627 Hannover (DE); HEUBLEIN, Nora, 30627 Hannover (DE); HEUBLEIN, Christoph, 30627 Hannover (DE)
(74) Vertreter: Eisenführ, Speiser & Partner
(86) Internationale Anmeldenummer: PCT/EP2003/012532
(87) Internationale Veröffentlichungsnummer: WO 2004/043474

(56) Entgegenhaltungen:
- EP-A- 0 518 061
- EP-A- 0 770 391
- WO-A-02/100452
- US-A- 4 610 241
- US-B1- 6 478 815
- BERND HEUBLEIN, ROLAND ROHDE, MATHIAS NIEMEYER, VOLKER KASSE ET AL.: "Degradation of Metallic Alloys - A new Principle in Stent Technology?" ABSTRACTS-ACCIS 2000, Februar 2000 (2000-02), Seiten 14A-15A, XP002291050 02-2000

## Beschreibung

Die Erfindung betrifft die medizinische Verwendung eines oder mehrerer der Elemente aus der Gruppe Yttrium, Neodym und Zirconium, pharmazeutische Formulierungen, die diese Elemente enthalten, sowie Implantate, die zumindest bereichsweise aus diesen Formulierungen aufgebaut sind.

Unter Entzündung wird die vom Bindegewebe und den Blutgefäßen getragene Reaktion des Organismus auf einen äußeren oder innerlich ausgelösten Entzündungsreiz mit dem Zweck, diesen zu beseitigen oder zu inaktivieren und die reizbedingte Gewebsschädigung zu reparieren verstanden. Auslösend wirken mechanische Reize (Fremdkörper, Druck, Verletzung) und andere physikalische Faktoren (ionisierende Strahlen, UV-Licht, Wärme, Kälte), chemische Stoffe (Laugen, Säuren, Schwermetalle, bakterielle Toxine, Allergene und Immunkomplexe) sowie Erreger (Mikroorganismen, Würmer, Insekten) beziehungsweise krankhafte Stoffwechselprodukte (entgleiste Enzyme, bösartige Tumore). Die durch die genannten auslösenden Faktoren komplexen mikrobiologischen Prozesse gehen in der Regel mit der Freisetzung sogenannter Wachstumsfaktoren wie FGF, PDGF und EGF einher, die die Proliferation, das heißt die Vermehrung von Gewebe durch Wucherung oder Sprossung, anregen.

Unter bestimmten medizinischen Indikationen sollte die Proliferation allerdings zumindest temporär gehemmt werden. Um der reproduktiven Aktivität der Zellen oder Organismen entgegenzuwirken ist es beispielsweise bekannt Mitosegifte, ionisierende Strahlen oder Interferone zur Virenbekämpfung einzusetzen.

Besondere Anforderungen bestehen bei der Behandlung von koronaren Herzerkrankungen. Koronare Herzerkrankungen, insbesondere akute Myokardinfarkte, stellen in Westeuropa und Nordamerika eine der häufigsten Todesursachen dar. In mehr als 80% der Fälle ist die Ursache des Myokardinfarktes der thrombotische Verschluss einer Koronararterie durch Ruptur einer atheromatösen Plaque bei vorbestehender stenosierender Atheromatose. Entscheidende Faktoren für die Langzeitprognose nach akutem Myokardinfarkt sind:
- eine effektive und langanhaltende Wiedereröffnung der Infarktarterie,
- die Dauer des thrombotischen Gefäßverschlusses,
- die Verhinderung eines größeren Myokardverlustes und eines ventrikulären Remodeling,
- die Beherrschung rhythmogener Komplikationen.

Die genannten Faktoren bestimmen nicht nur die kardiovaskuläre Mortalität, sondern auch die Lebensqualität nach dem Infarkt.

Seit mehr als zwanzig Jahren sind nicht-operative Methoden zur Stenose-Behandlung etabliert, bei denen u.a. durch Ballondilatation (PTCA Perkutane Transluminale Coronare Angioplastie) das verengte oder verschlossene Blutgefäß wieder aufgeweitet wird. Dieses Vorgehen hat sich insbesondere bei der Therapie des akuten Myokardinfarktes bewährt. Mit dem Aufweiten des Blutgefäßes entstehen allerdings kleinste Verletzungen, Einrisse, Dissektionen in der Gefäßwand, die zwar häufig problemlos verheilen, jedoch in etwa einem Drittel der Fälle durch das ausgelöste Zellwachstum zu Wucherungen führen (Proliferation), die letztendlich zu einer erneuten Gefäßverengung (Restenose) führen. Die Aufweitung beseitigt auch nicht die Ursachen der Stenose, also die molekularpathologischen Veränderungen in der Gefäßwand. Eine weitere Ursache der Restenose ist die Elastizität des gedehnten Blutgefäßes. Nach dem Entfernen des Ballons zieht sich das Blutgefäß übermäßig zusammen, so dass der Gefäßquerschnitt verringert wird (Obstruktion, sogenanntes negatives remodeling). Letzterer Effekt kann nur durch Platzierung eines Stents vermieden werden.

In der interventionellen Therapie der stabilen und instabilen Angina pectoris bei koronarer Herzkrankheit, hat die Einführung der Stents zu einer deutlichen Reduktion der Rate an Restenosen und damit zu besseren Langzeitresultaten geführt. Dies gilt sowohl für die primäre als auch die Rezidivstenose. Ursächlich für den Nutzen der Stent-Implantation ist der höhere primäre Lumengewinn.

Durch den Einsatz von Stents kann zwar ein optimaler Gefäßquerschnitt erreicht werden, allerdings führt der Einsatz von Stents ebenfalls zu kleinsten Verletzungen, die die Proliferation induzieren können und damit letztendlich eine Restenose auslösen können. Weiterhin initiiert die Anwesenheit eines derartigen Fremdkörpers eine Kaskade von zellulären molekularen Prozessen, die zu einem allmählichen Zuwachsen des Stents führen können.

Mittlerweile bestehen umfangreiche Erkenntnisse zum zellbiologischen Mechanismus und zu den auslösenden Faktoren der Stenose und Restenose. Die Restenose entsteht - wie bereits erläutert - als Reaktion der Gefäßwand auf die lokale Verletzung infolge der Dehnung des atherosklerotischen Plaque. Über komplexe Wirkmechanismen wird die lumengerichtete Migration und Proliferation der glatten Muskelzellen der Media und der Adventitia induziert (neointimale Hyperplasie). Unter Einfluss verschiedener Wachstumsfaktoren produzieren die glatten Muskelzellen eine Deckschicht aus neointimalen Glattmuskelzellen und Matrixproteinen (Elastin, Kollagen, Proteoglykane), deren ungesteuertes Wachstum allmählich zu einer Einengung des Lumens führen kann. Systemische medikamentöse Therapieeinsätze sehen u.a. die orale Verabreichung von Calzium-Antagonisten, ACE-Hemmern, Antikoagulantien, Antiaggregantien, Fischölen, antiproliferativen Substanzen, antiinflammatorischen Substanzen und Serotonin-Antagonisten vor, signifikante Reduktionen der Restenosearten wurden auf diesem Wege bisher jedoch nicht erreicht. Eine mögliche Erklärung für die enttäuschenden Ergebnisse aller bisherigen Versuche systemischer Applikation verschiedenster Substanzen ist darin zu sehen, dass eine systemische Applikation die Substanz nicht in ausreichender Konzentration an die Stelle der Gefäßverletzung bringen kann.

Seit einigen Jahren versucht man die Restenosegefahr bei der Implantation von Stents durch Aufbringung spezieller Beschichtungssysteme zu mindern. Teilweise dienen die Beschichtungssysteme als Träger, in die ein oder mehrere pharmakologisch wirksame Subtanzen eingebettet sind (Local Drug Delivery, LDD). Durch die lokale Applikation kann ein höherer Gewebsspiegel erreicht werden, wobei die systemische Substanzabgabe gering bleibt und damit die systemische Toxizität verringert wird. Die Beschichtungssysteme bedecken in der Regel zumindest eine der Gefäßwand zugewandte Umlaufswandung des endovaskulären Implantates. Als Wirkstoffe oder Wirkstoffkombinationen für LDD-Systeme wurden bisher zahlreiche Präparate vorgeschlagen, z. B. Paclitaxel, Actinomycin, Sirolimus, Tacrolimus, Everolimus und Dexamethason.

Der Träger derartiger Beschichtungssysteme besteht aus einem biokompatiblen Material, welches entweder natürlichen Ursprungs ist oder auf synthetischem Wege gewonnen werden kann. Eine besonders gute Verträglichkeit und die Möglichkeit, die Elutionscharakteristik des eingebetteten Arzneistoffs zu beeinflussen bieten biodegradierbare Beschichtungsmaterialien. Beispiele für die Verwendung biodegradierbarer Polymere sind Cellulose, Kollagen, Albumin, Casein, Polysaccharide (PSAC), Polylactid (PLA), Poly-L-lactid (PLLA), Polyglykol (PGA), Poly-D,L-lactid-co-glycolid (PDLLA/PGA), Polyhydroxybuttersäure (PHB), Polyhydroxyvaleriansäure (PHV), Polyalkylcarbonate, Polyorthoester, Polyethylenterephthalat (PET), Polymalonsäure (PML), Polyanhydride, Polyphosphazene, Polyaminosäuren und deren Copolymere sowie Hyaluronsäure und seine Derivate.

Derzeit werden 80% aller Stents aus medizinischem Stahl (316L) hergestellt. Im Laufe der Zeit zeigte sich allerdings, dass das eingesetzte Material zwar biokompatibel, aber über mittlere und lange Zeiträume teils eine Thrombosebildung und teils eine Adhäsion von Biomolekülen an ihrer Oberfläche förderten. Eine weitere Einschränkung der Biokompatibilität permanenter Stents ist die dauerhafte mechanische Reizung der Gefäßwand. Ein Ansatzpunkt zur Lösung dieser Problematik sind Stents aus einem biodegradierbaren Material. Unter Biodegradation werden hydrolytische, enzymatische und andere stoffwechselbedingte Abbauprozesse im lebendem Organismus verstanden, die zu einer allmählichen Auflösung zumindest großer Teile des Implantats führen. Synonym wird häufig der Begriff Biokorrosion verwendet. Der Begriff Bioresorption umfasst zusätzlich die anschließende Resorption der Abbauprodukte. So wurden beispielsweise eine Vielzahl von Kunststoffmaterialien als Stentwerkstoff vorgeschlagen, die zwar ein gutes Degradationsverhalten zeigten, jedoch aufgrund ihrer mechanischen Eigenschaften allenfalls eingeschränkt für die medizinische Applikation nutzbar sind und - so zumindest bei synthetischen Polymeren auf Basis von PU- und LDA-Derivaten - zudem starke inflammatorischer Reaktion hervorrufen und die Neointimaproliferation stimulieren.
Zur Überwindung vorgenannten Nachteils wird neuerdings der Einsatz spezieller biodegradierbarer Metalllegierungen vorgeschlagen, wie sie insbesondere in der DE 197 31 021 und DE 199 45 049 beschrieben werden. Die Metalllegierungen umfassen spezielle biodegradierbare Eisen-, Wolfram- und Magnesiumlegierungen.
Aus der US 6,264,595 ist ein Stent bekannt, der u.a. radioaktive Yttriumisotope enthalten kann, wobei die beim Zerfall der Isotope freigesetzte Strahlung die Restenose nach Stentimplantation verhindern soll. Die US 4,610,241 beschreibt ein Verfahren zur Behandlung von Atherosklerose mit ferro-, dia- oder paramagnetischen Partikeln, die nach Verbringung an den Ort der Läsion durch alternierende elektromagnetische Felder aufgeheizt werden. Die Partikel sollen u.a. bestimmte Yttriumsalze umfassen.
Zirconium ist Bestandteil zahlreicher keramischer Biomaterialien. Bisherige in vivo und in vitro Untersuchungen an speziellen zirconiumhaltigen Keramiken liefern keine Hinweise auf eine pharmakologische Wirkung in Zusammenhang mit glatten humanen Muskelzellen (Piconi, C, Maccauro G., (1999) Biomaterials 20, 1-25).
EP 0 770 391 A1 beschreibt pharmazeutische Formulierungen, in denen Yttrium- und Neodymsalze als Antagonisten einer Substanz P, die auf glatte Muskelzellen kontraktorisch wirkt, eingesetzt werden.
Der vorliegenden Erfindung liegt u.a. die Aufgabe zugrunde die Proliferation von humanen glatten Muskelzellen hemmende Mittel und pharmazeutische Formulierungen bereit zu stellen, die sich insbesondere für den Einsatz in endovaskuläre Implantate wie Stents eignen.
Gemäß einem ersten Aspekt der Erfindung wird die gestellte Aufgabe gelöst durch Verwendung eines oder mehrerer der Elemente aus der Gruppe Yttrium (Y), Neodym (Nd) oder Zirconium (Zr) zur Herstellung einer die Proliferation von humanen glatten Muskelzellen hemmenden pharmazeutischen Formulierung.

Es hat sich nun überraschenderweise gezeigt, dass die Proliferation von humanen glatten Muskelzellen, insbesondere arteriellen Muskelzellen, in Gegenwart von Yttrium, Neodym und/oder Zirconium deutlich gehemmt wird. Insbesondere kann durch Einsatz dieser Elemente die neointimale Hyperplasie nach Ballondilatation vermindert oder gar gänzlich verhindert werden. Besondere geeignet erscheint die Verwendung eines oder mehrerer der Elemente aus der Gruppe Yttrium, Neodym, oder Zirconium zur Behandlung sklerotischer, vorzugsweise atherosklerotischer Läsionen. Bei den die Restenose begründenden pathophysiologischen Prozessen spielt die Proliferation von zuvor aus der Media migrierten glatten Muskelzellen eine entscheidende Rolle. Eine Hemmung des Zellwachstums über einen bestimmten Zeitraum bis die das Wachstum stimulierenden Faktoren größtenteils oder vollständig abgebaut sind kann daher einer Restenose wirksam vorbeugen. Die Elemente Yttrium, Neodym und/oder Zirconium eignen sich somit insbesondere zur Restenoseprophylaxe nach Stentimplantation. Die Gründe für die überraschende pharmazeutische Wirkung der Elemente Yttrium, Zirconium und/oder Neodym auf humane arterielle glatte Muskelzellen sind noch nicht gänzlich geklärt. Vermutlich spielen die im Zellmedium unter Anteilnahme der Metalle stattfindenden Redoxprozesse eine wesentliche Rolle.

Bisherige in vivo und in vitro Untersuchungen an Säugetieren und Fischen hinsichtlich der toxischen und möglicherweise pharmazeutischen Wirkung von Yttriumtrichlorid (YCl₃) liefern keine Hinweise auf die besondere pharmazeutische Wirkung von Yttrium auf arterielle humane glatte Muskelzellen.
- Bei intravenöser Gabe von 1 mg YCl₃/107g-Ratte wurde im Blutplasma 20 Stunden nach Zugabe eine Erhöhung der Aspartat- und Glutamat-Pyruvat-Transaminase Aktivität gemessen, welches auf eine Leber-Schädigung hinweist (Hirano, S., Kodama, N., Shibata, K., and Suzuki, K. T. (1993) Toxicol Appl Pharmacol 121(2), 224-232).
- Intratracheal appliziertes YCl₃ führt zu einer Aktivierung der Immunantwort in der Lunge (Hirano, S., Kodama, N., Shibata, K., and Suzuki, K. T. (1990) Toxicol Appl Pharmacol 104(2), 301-311) und zu einem Anstieg inflammatorischer Marker
- (β-Glucuronidase, Lactat-Dehydrogenase (LDH) und Alkalische Phosphatase) im *bronchoalveolar lavage fluid* (BALF) (Suzuki, K. T., Kobayashi, E., Ito, Y., Ozawa, H., and Suzuki, E. (1992) Toxicology 76(2), 141-152; Marubashi, K., Hirano, S., and Suzuki, K. T. (1998) Toxicol Lett 99(1), 43-51).
- Eine Konzentration von 15µM YCl₃ im Wasser führt zu einer Senkung der Superoxid-Dismutase-Aktivität in der Goldfisch-Leber, während die Catalase-Aktivität nur geringfügig beeinträchtigt wird (Chen, Y., Cao, X. D., Lu, Y., and Wang, X. R. (2000) Bull Environ Contam Toxicol 65(3), 357-365).
- Mit der "isolierten Organ Technik" wurde gezeigt, dass YCl₃ die Amplitude der peristaltischen Aktivität des Ratten-Darms senkt (Cunat, L., Membre, H., Marchal, L., Chaussidon, M., and Burnel, D. (1998) Biol Trace Elem Res 64(1-3), 43-59).
- In vitro wurde eine genotoxische Wirkung von YCl₃ auf humane Lymphozyten nachgewiesen (Yang, H., Ji, Q., and Zhang, X. (1998) Zhonghua Yu Fang Yi Xue Za Zhi 32(3), 156-158).
- YCl₃ blockiert Ca²⁺-Kanäle *in vitro* (Beedle, A. M., Hamid, J., and Zamponi, G. W. (2002) J Membr Biol 187(3), 225-238; Mlinar, B., and Enyeart, J. J. (1993) J Physiol 469, 639-652).

Weiterhin gibt es Studien über den Einfluss von Yttrium auf die Proliferation von Bakterien. Untersucht wurden *Tetrahymena shanghaiensis* (Wang, Y., Zhang, M., and Wang, X. (2000) Biol Trace Elem Res 75(1-3), 265-275), *Klebsiella pneumoniae* Stamm 204 und K9 (Aleksakhina, N. N., Miriasova, L. V., and Basnak'ian, I. A. (2002) Zh Mikrobiol Epidemiol Immunobiol (6), 13-18) sowie *Pseudomonas fluorescens* (Appanna, V. D., Hamel, R. D., Pankar, E., and Puiseux-Dao, S. (2001) Microbios 106(413), 19-29). Dabei zeigte sich eine erhöhte Proliferation für *T. shanghaiensis* und *P. fluorescens* bei niedrigen Yttrium-Konzentrationen und eine antiproliferative Wirkung bei hohen Konzentrationen. Für *K. pneumoniae* wurde eine vergleichsweise hohe Konzentration (142mM Y(OH)₃) getestet, die zu einer erhöhten Proliferation führte.

Ein zweiter Aspekt der Erfindung betrifft pharmazeutische Formulierungen, die eines oder mehrere der Elemente aus der Gruppe Yttrium, Neodym oder Zirconium als einen die Proliferation von humanen glatten Muskelzellen hemmenden Bestandteil enthalten wobei die Formulierung zur intravaskulären Freisetzung nach Implantation in ein vaskuläres Gefäß angepasst ist und einen zumindest weitgehend biodegradierbaren Träger umfasst, der in vivo mit einem vorbestimmten Degradationsverhalten abgebaut wird.

Unter dem Begriff "Degradationsverhalten" wird der über die Zeit durch chemische, thermische, oxidative, mechanische oder biologische Prozesse stattfindende Abbau des Trägers im lebenden Organismus verstanden. Dieser Aspekt der Erfindung hat demnach dann Bedeutung, wenn die Formulierung für die intravaskuläre Freisetzung nach Implantation in ein vaskuläres Gefäß anzupassen ist. Insbesondere soll eine lokale Applikation der Wirkstoffe im Bereich der zu behandelnden Läsion erfolgen. Derartige Ansätze lassen sich unter dem Begriff 'local drug delivery' (LDD) zusammenfassen.
Nach einer bevorzugten Variante ist der biodegradierbare Träger eine Legierung, insbesondere eine Magnesium-, Eisen- oder Wolframlegierung. Derartige Metalllegierungen sind beispielsweise aus der DE 197 31 021 und DE 199 45 049 bekannt. Eine weitere, besonders geeignete Formulierung auf Basis einer Magnesiumlegierung hat folgende Zusammensetzung:
Magnesium: > 90 %
Yttrium: 3,7 % bis 5,5 %
Seltene Erden (ohne Yttrium): 1,5 % bis 4,4 %
Rest: < 1%.
Vorzugsweise umfasst die Formulierung ferner eine Magnesiumlegierung mit einem Gehalt von Yttrium im Bereich von 3,7 bis 5,5 Gew.%, einem Gehalt von Neodym im Bereich von 1,8 bis 2,7 Gew.% und einem Gehalt von Zirconium im Bereich von 0,2 bis 1,2 Gew.%. Besonders bevorzugt entspricht die Formulierung der kommerziell erhältlichen Magnesiumlegierung WE43 (W-25 EP 5M). Die vorgenannten Materialien und Angaben zur Zusammensetzung zeichnen sich durch ihre gute Verarbeitbarkeit und günstiges Freisetzungsverhalten für Yttrium, Neodym und Zirconium beim in vivo-Abbau des Trägers aus. Aus der Literatur ist u.a. eine Studie zum Degradationsverhalten einer Magnesiumlegierung unter physiologischen Bedingungen bekannt, die Hinweise dazu liefert, welche Faktoren und Maßnahmen bei der Optimierung der Wirkstofffreisetzung zu beachten sind (Levesque, J., Dube, D., Fiset M. and Mantovani, D. (2003) Material Science Forum Vols. 426-432, pp. 225-238).

Nach einer weiteren Variante der erfindungsgemäßen Formulierung ist der Träger ein biodegradierbares Polymer und eines oder mehrere der Elemente aus der Gruppe Yttrium, Neodym oder Zirconium wird in Form von Pulvern oder Mikropartikeln in das Polymer eingebettet. Durch den allmählichen Abbau des Polymers in vivo wird das Pulver beziehungsweise die Mikropartikel langsam freigesetzt und können nach Bioresorption ihre pharmakologische Wirkung entfalten. Der polymere Träger kann insbesondere Hyaluronsäure, Poly-L-Lactid oder ein Derivat der Polymere sein.

Weiterhin ist bevorzugt, wenn die Formulierung Yttrium in einem Mengenanteil von 0,1 bis 10 Gew.%, Neodym in einem Mengenanteil von 0,1 bis 5 Gew.% und/oder Zirconium in einem Mengenanteil von 0,1 bis 3 Gew.%, jeweils bezogen auf das Gesamtgewicht der Formulierung, enthält.

Aus den Zellkulturversuchen ist bekannt, dass die Elemente der Gruppe Yttrium, Neodym und Zirconium in bestimmten Konzentrationsbereichen ein antiproliferatives Verhalten auf arterielle humane glatte Muskelzellen zeigen. Die erfindungsgemäße Formulierung wird daher, sofern sie Yttrium enthält, derart angepasst, dass eine Yttriumkonzentration im Bereich der zu behandelnden humanen glatten Muskelzellen zwischen 200 µM bis 2 mM, insbesondere zwischen 800 bis 1 mM, liegt. Enthält die Zusammensetzung Neodym, so wird die Formulierung vorzugsweise derart angepasst, dass eine Neodymkonzentration im Bereich der zu behandelnden humanen glatten Muskelzellen zwischen 600 µM bis 2 mM, insbesondere zwischen 800 µM bis 1 mM liegt. Ist Zirconium Bestandteil der Formulierung, so ist vorzugsweise eine Zirconiumkonzentration im Bereich der zu behandelnden humanen glatten Muskelzellen durch gezielte Anpassung der Formulierung zwischen 200 µM bis 2 mM, insbesondere zwischen 200 µM bis 1 mM, vorzugeben. Besonders bevorzugt ist es bei einer Formulierung, die Yttrium, Neodym und Zirkonium enthält, die Formulierung derart anzupassen, dass eine Yttriumkonzentration bei 350 bis 550 µM, eine Neodymkonzentration bei 100 bis 200 µM und eine Zirconiumkonzentration bei 10 bis 30 µM im Bereich der zu behandelnden humanen glatten Muskelzellen liegt. Die genannten Konzentrationsbereiche erscheinen besonders geeignet für eine Restenoseprophylaxe nach Stentimplantation, da die systemische Substanzabgabe sehr gering ist und daher mit allenfalls einer geringen systemischen Toxizität gerechnet werden muss.

Die tatsächlichen Konzentrationen im lebenden Organismus sind abhängig vom Degradationsverhalten der Formulierung, die wiederum von der konkreten Zusammensetzung der Formulierung abhängt, und dem Diffusionsverhalten der Abbauprodukte im Gewebe. Theoretische Vorhersagen sind hier nur schwer möglich und entsprechende Messungen sind häufig mit großen Messfehlern behaftet. Damit sich die vorgenannten Konzentrationsbereiche in der Umgebung der zu behandelnden humanen glatten Muskelzellen einstellen, sind daher in der Regel noch experimentelle Studien zur Bioresorption der gewählten Formulierung notwendig.

Durch eigene Experimente ist u.a. eine statistisch signifikante Reduktion der Neointimabildung in Schweinen bei Verwendung der Legierung WE43 und dem damit gegebenen Degradationsverhalten (weitgehende Biodegradation innerhalb von 2 Monaten) belegt. Die dort eingesetzten Koronarstents hatten ein Gewicht von 3 mg und enthielten 123 µg Yttrium (4,1 Gew.%), 66 µg Neodym (2,2 Gew.%) und 15 µg Zirconium (0,5 Gew.%).

Ein dritter Aspekt der Erfindung betrifft Implantate, die eine zumindest bereichsweise Beschichtung aus der vorgenannten erfindungsgemäßen Formulierung aufweisen oder die strukturell in Teilen aus dieser Formulierung bestehen. Ein solches Implantat kann vorzugsweise als endovaskuläres Stützgerüst (Stent) ausgebildet sein.
Eine Verteilung und Masse der Formulierung in einem Stent wird vorzugsweise derart bezogen auf die Länge des Stents vorgegeben, dass ca. 5 bis 30 µg/mm, insbesondere 10 bis 20 µg/mm, Yttrium vorhanden sind. Für Neodym wird dieser Maßgabe vorzugsweise auf ca. 2 bis 20 µg/mm, insbesondere 3 bis 10 µg/mm, und für Zirconium vorzugsweise auf ca. 0,05 bis 10 µg/mm, insbesondere 0,5 bis 6 µg/mm, festgelegt. Die genannten Bereichgrenzen erlauben eine pharmakodynamisch günstige lokale Applikation der Wirkstoffe.

Nachfolgend wird die Erfindung in Ausführungsbeispielen und anhand von dazugehörigen Zeichnungen näher erläutert. Es zeigen:
- Figur 1: eine schematische Darstellung einer Endoprothese in Form eines Stents,
- Figur 2: einen typischen Schnitt durch ein Haupt-Koronargefäß des Schweins nach Implantation eines herkömmlichen Stents und
- Figur 3: einen typischen Schnitt durch ein Haupt-Koronargefäß des Schweins nach Implantation eines Stent aus dem Werkstoff WE43.

### Testung von Yttriumchlorid (YCl₃), Zirconiumchlorid (ZrCl₄) und Neodymiumchlorid (NdCl₃) in Zellkultur

Es wurden jeweils für Yttrium, Neodym und Zirconium Testreihen an arteriellen humanen glatten Muskelzellen mit Konzentrationen im Bereich von 1 mM bis 1 µM wie folgt durchgeführt:
Es wurde die Wirkung von YCl₃x6H₂O, ZrCl₄ und NdCl₃ auf die Vitalität und Proliferation humaner arterieller glatter Muskelzellen (SMC) untersucht. Es ist davon auszugehen, dass die Elemente in physiologischer Umgebung oxidiert werden und eine Bioresorption der Seltenerden-Ionen Y³⁺, Zr⁴⁺ und Nd³⁺ stattfindet. Getestet wurde in Konzentrationsbereichen von 1 mM bis 1 µM, jeweils bezogen auf den Gehalt an Seltenerden. Niedrigere Konzentrationen zeigten keine Effekte.
Die Substanzen wurden in Wasser bzw. Ethanol (ZrCl₄) gelöst (Stammlösung 0,1 M, jeweils bezogen auf die Konzentration der Seltenerden). Beim Verdünnen in Zellkulturmedium bildete sich bei höheren Konzentrationen Niederschlage, die durch Ultraschallbehandlung reduziert, aber nicht vollständig eliminiert werden konnten. Die hergestellten Eluate wurden mit Primärzell-Kulturen humaner arterieller glatter Muskelzellen (SMC) inkubiert (3 Tage, 37° C). Es wurde die Zellvitalität (MTS-Test) sowie die Zellproliferation (BrdU-Test) untersucht. Dazu wurden Tests analog einer Zytotoxizitätsprüfung nach DIN EN 30993-5 durchgeführt.
Die Vitalität arterieller humaner glatter Muskelzellen stieg in dem Konzentrationsbereich von 1 µM bis 100 µM an. Konzentrationen von > 800 µM an Neodym und Zirconium führten zu einem Vitalitätsabfall.
Die Proliferation arterieller humaner glatter Muskelzellen wurde bei Neodymkonzentrationen ≥ 800 µM zunehmend stark inhibiert. Bereits weitgehende Inhibition der Proliferation war bei Yttriumkonzentrationen von ≥ 800 µM festzustellen. Bei Zirconiumkonzentrationen von 200 µM bis 1 mM lag die Proliferation durchschnittlich bei 44%. Somit zeigten Yttrium und Neodym bei höheren Konzentrationen eine starke Wirkung auf die Proliferation glatter Muskelzellen. Zirconium hatte eine moderate antiproliferative Wirkung.

### Testung von WE43-Eluaten in Zellkultur

Sterilisierte Probenkörper der Legierung WE 43 mit einem Gewicht von ca. 1 mg wurden mit 2 ml Zellkulturmedium bei 37°C im Zellkulturschrank für 13 Tage eluiert, wobei der Probenkörper nur unvollständig in Lösung geht. Anschließend wurden Primärzell-Kulturen humaner arterieller glatter Muskelzellen (SMC) mit 1 ml des Eluats und 1 ml frischen Zellkulturmedium inkubiert (4 Tage, 37° C). Es wurde die Zellvitalität (MTS-Test) sowie die Zellproliferation (BrdU-Test) untersucht. Dazu wurden Tests analog einer Zytotoxizitätsprüfung nach DIN EN 30993-5 durchgeführt.

Die Proliferation glatter Muskelzellen wurde bei Inkubation mit Eluaten der Legierung WE43 im Vergleich zu Kontrollzellen (SMC + Medium) zu 91% inhibiert. Die Zellvitalität der glatten Muskelzellen für die Legierung WE43 lag bei 95%.

### Tierversuche am Schwein

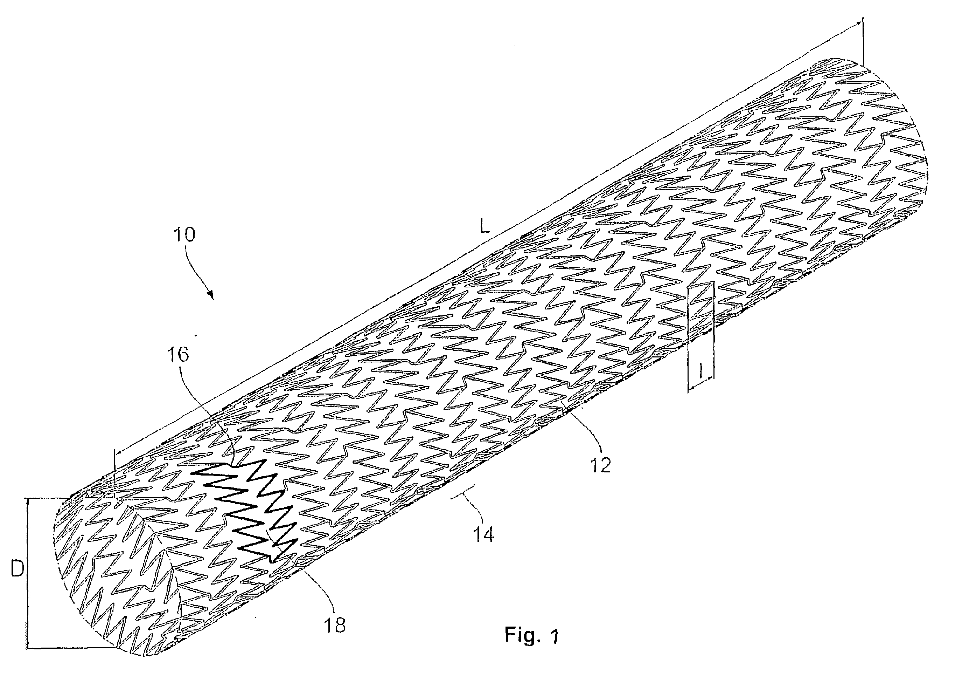

Figur 1 zeigt eine vaskuläre Endoprothese in Form eines rohrförmigen Stents 10, dessen Grundgerüst aus einer Vielzahl einzelner Stege 12 zusammengesetzt ist. Das Grundgerüst des Stents 10 lässt sich in Längsrichtung in einzelne Stützabschnitte 14 gliedern, die sich jeweils aus ein zick-zack- oder mäanderförmig gefalteten und in Umfangrichtung verlaufenden Stegen 12 zusammensetzen. Das Grundgerüst des Stents 10 wird von mehreren solcher in Längsrichtung aufeinanderfolgender Stützabschnitte 14 gebildet. Die Stützabschnitte 14 sind über Verbindungsstege 16 miteinander verbunden. Jeweils zwei in Umfangsrichtung aneinander benachbarte Verbindungsstege 16 sowie die zwischen diesen Verbindungsstegen 16 einander gegenüberliegenden Teilabschnitte der Stützabschnitte 14 definieren eine Masche 18 des Stents 10. Eine solche Masche 18 ist in Figur 1 hervorgehoben dargestellt. Jede Masche 18 umschließt eine radiale Öffnung der Umfangswandung bzw. des Grundgerüsts des Stents 10.
Jeder Stützabschnitt 14 weist etwa drei bis sechs über den Umfang des Stents 10 gleich verteilte Verbindungsstege 16 auf, die jeweils einen Stützabschnitt 14 mit dem benachbarten Stützabschnitt 14 verbinden. Dementsprechend weist der Stent 10 in Umfangsrichtung zwischen zwei Stützabschnitten 14 jeweils drei bis sechs Maschen auf.
Aufgrund der Faltung der Stege 12 ist der Stent 10 in Umfangsrichtung expandierbar. Dies geschieht beispielsweise mit einem an sich bekannten und hier nicht dargestellten Ballonkatheter, der an seinem distalen Ende einen mittels eines Fluids expandierbaren Ballon aufweist. Der Stent 10 ist im komprimierten Zustand auf den deflatierten Ballon aufgecrimpt. Mit Expansion des Ballons werden sowohl der Ballon als auch der Stent 10 aufgeweitet. Anschließend kann der Ballon wieder deflatiert werden und der Stent 10 löst sich von dem Ballon. Auf diese Weise kann der Katheter gleichzeitig dem Einführen des Stents 10 in ein Blutgefäß und insbesondere in ein verengtes Herzkranzgefäß sowie zum Expandieren des Stents an diesem Ort dienen.

Das Grundgerüst des in Figur 1 dargestellten Stents 10 besteht aus der biodegradier-baren Magnesiumlegierung WE43 mit folgender Formulierung:
Zirconium: 0,53 Gew.%
Yttrium: 4,1 Gew.%
Neodym: 2,2 Gew.%
Andere: <0,4 Gew.%
Magnesium: Rest auf 100 Gew.%.

Nimmt man für einen 10 mm langen Stent aus WE 43 das Gewicht von 3 mg an, enthält er ca. 123 µg /1.384 µM Yttrium (4,1 Gew.%), ca. 66 µg / 458 µM Neodym (2,2 Gew.%) und ca. 15 µg / 164 µM Zirconium (0,5 Gew.%). Pro mm Stentlänge ergibt sich eine maximale Freisetzung von 12,3 µg /138,4 µM Yttrium, 6,6 µg / 45,8 µM Neodym und 1,5 µg / 16,4 µM Zirconium.

In Tierversuchen am Schwein wurden Stents aus vorgenannter Magnesiumlegierung mit herkömmlichen Siliziumkarbid beschichteten Stents mittels Koronarangiographie und morphometrischer Auswertung histologischer Schnittpräparate verglichen. Dazu wurden herkömmliche Stents aus medizinischem Edelstahl mit einer passiven Siliziumkarbidbeschichtung und Stents aus WE43 in alle drei Koronarien von Schweinen implantiert. Nach vier und acht Wochen erfolgte jeweils eine quantitative Kontrollangiographie (QCA), wobei der Abbau beim biodegradierbaren Stent im Schwein nach etwa 8 Wochen weitestgehend abgeschlossen war. Nach 8 Wochen wurden zudem Herzpräparate der Tiere zur histologischen Aufarbeitung angefertigt.

Die Ergebnisse der Koronarangiographie und histologischen Schnittpräparate belegen einen deutlichen Trend hin zu einer Verringerung der Flächenstenose bei Einsatz von WE43. Die Histologie zeigte ein weitgehend einheitliches Bild in bezug auf die Neointimabildung nach acht Wochen. Dabei erwiesen sich die Magnesiumimplantate als weniger proliferativ als die Kontrollimplantate. So wurde eine durchschnittliche Neointima-flächenbildung von 1,23 mm² bei Einsatz von WE43 im Vergleich zu 2,9 mm² bei einem herkömmlichen Implantat festgestellt.

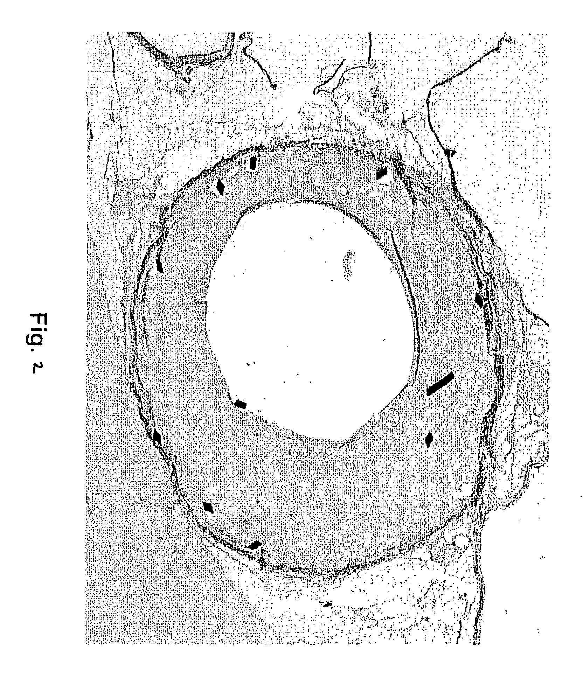

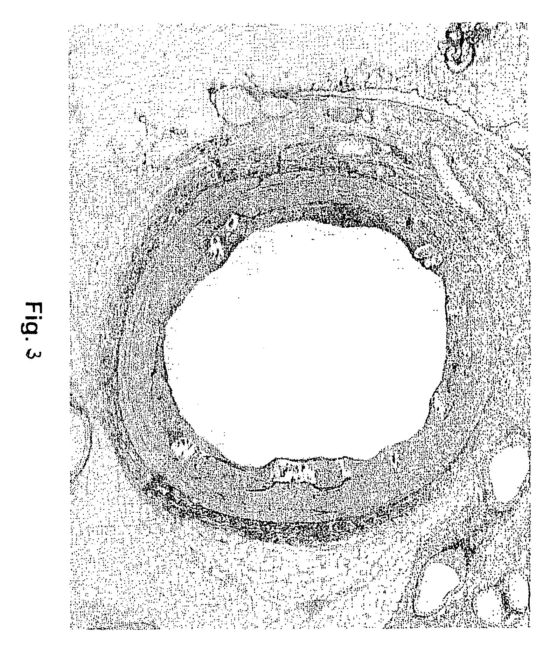

In der Figur 2 ist ein typischer Schnitt durch ein Koronargefäß eines Schweins bei Implantation eines herkömmlichen Stents mit Siliziumkarbidbeschichtung nach acht Wochen dargestellt, während Figur 3 einen entsprechenden histologischen Schnitt für ein Implantat auf Basis von WE43 wiedergibt. Deutlich wird, dass Neointimabildung, die durch den morphometrischen Querschnitt der Neointimaflächen nach acht Wochen abschätzbar ist, etwa um den Faktor 2 bei Einsatz von WE43 gemindert ist. Der Effekt scheint im wesentlichen durch die bei der Degradation des Stents in die Gewebsumgebung freigesetzten Rückstände bedingt zu sein, die wiederum Yttrium, Neodym und Zirconium beinhalten.

## Patentansprüche

1. Verwendung eines oder mehrerer der Elemente aus der Gruppe Yttrium (Y), Neodym (Nd) oder Zirconium (Zr) zur Herstellung einer die Proliferation von humanen glatten Muskelzellen hemmenden pharmazeutischen Formulierung.

2. Verwendung der pharmazeutischen Formulierung nach Anspruch 1 zur Hemmung der Proliferation humaner glatter Muskelzellen im Bereich sklerotischer, insbesondere atherosklerotischer Läsionen.

3. Verwendung nach den Ansprüchen 1 oder 2 zur lokalen Restenoseprophylaxe nach Stentimplantation.

4. Pharmazeutische Formulierung, enthaltend die Elemente Yttrium (Y), Neodym (Nd) und Zirconium (Zr) als die Proliferation von humanen, glatten Muskelzellen hemmende Bestandteile

5. Formulierung nach Anspruch 4, **dadurch gekennzeichnet, dass** der Träger eine Legierung, insbesondere Magnesium-, Eisen- oder Wolframlegierung, ist.

6. Formulierung nach Anspruch 4, **dadurch gekennzeichnet, dass** der Träger ein bioresorbierbares Polymer ist und eines oder mehrere der Elemente aus der Gruppe Y, Nd oder Zr in Form von Pulvern oder Mikropartikeln in das Polymer eingebettet sind.

7. Formulierung nach einem der vorhergehenden Ansprüche, **dadurch gekennzeichnet, dass** die Formulierung Y in einem Mengenanteil von 0,1 bis 10 Gew.%, bezogen auf das Gesamtgewicht der Formulierung, enthält.

8. Formulierung nach einem der vorhergehenden Ansprüche, **dadurch gekennzeichnet, dass** die Formulierung Nd in einem Mengenanteil von 0,1 bis 5 Gew.%, bezogen auf das Gesamtgewicht der Formulierung, enthält.

9. Formulierung nach einem der vorhergehenden Ansprüche, **dadurch gekennzeichnet, dass** die Formulierung Zr in einem Mengenanteil von 0,1 bis 3 Gew.%, bezogen auf das Gesamtgewicht der Formulierung, enthält.

10. Formulierung nach Anspruch 5, **dadurch gekennzeichnet, dass** die die Formulierung eine Magnesiumlegierung ist und Y im Bereich von 3,7 bis 5,5 Gew.%, Seltene Erden ohne Y im Bereich von 1,5 bis 4,4 Gew.% und Restelemente < 1 Gew.% enthält.

11. Formulierung nach Anspruch 5, **dadurch gekennzeichnet, dass** die die Formulierung eine Magnesiumlegierung ist und Y im Bereich von 3,7 bis 5,5 Gew.%, Nd im Bereich von 1,8 bis 2,7 Gew.% und Zr im Bereich von 0,2 bis 1,2 Gew.% enthält.

12. Formulierung nach Anspruch 11, **dadurch gekennzeichnet, dass** die Magnesiumlegierung WE43 (W25/EP5M) ist.

13. Formulierung nach einem der vorhergehenden Ansprüche, **dadurch gekennzeichnet, dass** die Formulierung Y enthält und derart angepasst ist, dass eine Yttriumkonzentration im Bereich der zu behandelnden humanen glatten Muskelzellen zwischen 200 µM bis 2 mM, insbesondere zwischen 800 µM bis 1 mM, liegt.

14. Formulierung nach einem der vorhergehenden Ansprüche, **dadurch gekennzeichnet, dass** die Formulierung Nd enthält und derart angepasst ist, dass eine Neodymkonzentration im Bereich der zu behandelnden glatten Muskelzellen zwischen 600 µM bis 2 mM, insbesondere zwischen 800 µM bis 1 mM, liegt.

15. Formulierung nach einem der vorhergehenden Ansprüche, **dadurch gekennzeichnet, dass** die Formulierung Zr enthält und derart angepasst ist, dass eine Zirconiumkonzentration im Bereich der zu behandelnden glatten Muskelzellen zwischen 200 µM bis 2 mM, insbesondere zwischen 200 µM bis 1 mM, für liegt.

16. Formulierung nach einem der Ansprüche 1 bis 12, **dadurch gekennzeichnet, dass** die Formulierung Y, Nd und Zr enthält und angepasst ist eine Yttriumkonzentration zwischen 350 bis 550 µM, eine Neodymkonzentration zwischen 100 bis 200 µM und eine Zirconiumkonzentration zwischen 10 bis 30 µM im Bereich der zu behandelnden glatten Muskelzellen liegt.

17. Implantat mit einer Beschichtung oder einem Bestandteil aus einer Formulierung nach einem der Ansprüche 4 bis 16.

18. Implantat nach Anspruch 17, **dadurch gekennzeichnet, dass** das Implantat ein endovaskuläres Stützgerüst, insbesondere ein Stent ist.

19. Implantat nach Anspruch 18, **dadurch gekennzeichnet, dass** ca. 5 bis 30 µg Yttrium, insbesondere 10 bis 20 µg Yttrium, bezogen auf 1 mm Stentlänge, vorhanden sind.

20. Implantat nach Anspruch 18, **dadurch gekennzeichnet, dass** ca. 2 bis 20 µg Neodym, insbesondere 3 bis 10 µg Neodym, bezogen auf 1 mm Stentlänge, vorhanden sind.

21. Implantat nach Anspruch 18, **dadurch gekennzeichnet, dass** ca. 0,05 bis 10 µg Zirconium, insbesondere 0,5 bis 6 µg Zirconium, bezogen auf 1 mm Stentlänge, vorhanden sind.

## Claims

1. A use of one or more elements from the group yttrium (Y), neodymium (Nd), or zirconium (Zr) for producing a pharmaceutical formulation which inhibits the proliferation of human smooth muscle cells.

2. The use of the pharmaceutical formulation from Claim 1 for inhibiting the proliferation of human smooth muscle cells in the area of sclerotic, in particular arteriosclerotic lesions.

3. The use according to Claims 1 or 2 for local restenosis prophylaxis after stent implantation.

4. A pharmaceutical formation, containing the elements yttrium (Y), neodymium (Nd), and zirconium (Zr) as components which inhibit the proliferation of human smooth muscle cells.

5. The formulation according to Claim 4, **characterized in that** the carrier is an alloy, in particular a magnesium, iron, or tungsten alloy.

6. The formulation according to Claim 4, **characterized in that** the carrier is a bioresorbable polymer and one or more of the elements from the group yttrium, neodymium, or zirconium are embedded in the polymer in the form of powders or microparticles.

7. The formulation according to one of the preceding claims, **characterized in that** the formulation contains yttrium in an amount proportion of 0.1 to 10 wt.-%, in relation to the total weight of the formulation.

8. The formulation according to one of the preceding claims, **characterized in that** the formulation contains neodymium in an amount proportion of 0.1 to 5 wt.-%, in relation to the total weight of the formulation.

9. The formulation according to one of the preceding claims, **characterized in that** the formulation contains zirconium in an amount proportion of 0.1 to 3 wt.-%, in relation to the total weight of the formulation.

10. The formulation according to Claim 5, **characterized in that** the formulation is a magnesium alloy and contains yttrium in the range from 3.7 to 5.5 wt.-%, rare earth elements without yttrium in the range from 1.5 to 4.4 wt.-%, and residual elements < 1 wt.-%.

11. The formulation according to Claim 5, **characterized in that** the formulation is a magnesium alloy and contains yttrium in the range from 3.7 to 5.5 wt.-%, neodymium in the range from 1.8 to 2.7 wt.-%, and zirconium in the range from 0.2 to 1.2 wt.-%.

12. The formulation according to Claim 11, **characterized in that** the magnesium alloy is WE43 (W25/EP5 M).

13. The formulation according to one of the preceding claims, **characterized in that** the formulation contains yttrium and is tailored in such a way that an yttrium concentration between 200 µM and 2 mM, in particular between 800 µM and 1 mM, is provided in the area of the human smooth muscle cells to be treated.

14. The formulation according to one of the preceding claims, **characterized in that** the formulation contains neodymium and is tailored in such a way that a neodymium concentration in the range between 600 µM and 2 mM, in particular between 800 µM and 1 mM, is provided in the area of the smooth muscle cells to be treated.

15. The formulation according to one of the preceding claims, **characterized in that** the formulation contains zirconium and is tailored in such a way that a zirconium concentration between 200 µM and 2 mM, in particular between 200 µM and 1 mM, is provided in the area of the smooth muscle cells to be treated.

16. The formulation according to one of Claims 1 through 12, **characterized in that** the formulation contains yttrium, neodymium, and zirconium, and is tailored in such a way that an yttrium concentration between 350 and 550 µM, a neodymium concentration between 100 and 200 µM, and a zirconium concentration between 10 and 30 µM are provided in the area of the smooth muscle cells to be treated.

17. An implant having a coating or a component made of a formulation according to one of Claims 4 through 16.

18. The implant according to Claim 17, **characterized in that** the implant is an endovascular support frame, in particular a stent.

19. The implant according to Claim 18, **characterized in that** approximately 5 to 30 µg yttrium, in particular 10 to 20 µg yttrium, is provided in relation to 1 mm stent length.

20. The implant according to Claim 18, **characterized in that** approximately 2 to 20 µg neodymium, in particular 3 to 10 µg neodymium, is provided in relation to 1 mm stent length.

21. The implant according to Claim 18, **characterized in that** approximately 0.05 to 10 µg zirconium, in particular 0.5 to 6 µg zirconium, is provided in relation to 1 mm stent length.

## Revendications

1. Utilisation d'un ou de plusieurs des éléments du groupe d'yttrium (Y), néodyme (Nd) ou zirconium (Zr) pour la fabrication d'une formulation pharmaceutique inhibant la prolifération de cellules musculaires lisses humaines.

2. Utilisation de la formulation pharmaceutique selon la revendication 1 pour inhiber la prolifération de cellules musculaires lisses humaines dans la zone de lésions de sclérose, en particulier d'athérosclérose.

3. Utilisation selon les revendications 1 ou 2 pour la prophylaxie locale de la resténose après l'implantation de stent.

4. Formulation pharmaceutique, contenant les éléments yttrium, (Y), néodyme (Nd) et zirconium (Zr) en tant que composants inhibant la prolifération de cellules musculaire humaines lisses.

5. Formulation selon la revendication 4, **caractérisée en ce que** le support est un alliage, en particulier un alliage de magnésium, de fer ou de tungstène.

6. Formulation selon la revendication 4, **caractérisée en ce que** le support est un polymère biorésorbable et un ou plusieurs des éléments du groupe Y, Nd ou Zr sont intégrés sous forme de poudres ou de microparticules dans le polymère.

7. Formulation selon l'une quelconque des revendications précédentes, **caractérisée en ce que** la formulation contient du Y dans une fraction de quantité de 0,1 à 10 % en poids par rapport au poids total de la formulation.

8. Formulation selon l'une quelconque des revendications précédentes, **caractérisée en ce que** la formulation contient du Nd dans une fraction de quantité de 0,1 à 5 % en poids par rapport au poids total de la formulation.

9. Formulation selon l'une quelconque des revendications précédentes, **caractérisée en ce que** la formulation contient du Zr dans une fraction de quantité de 0,1 à 3 % en poids par rapport au poids total de la formulation.

10. Formulation selon la revendication 5, **caractérisée en ce que** la formulation est un alliage de magnésium et contient du Y dans la plage de 3,7 à 5,5 % en poids, des terres rares sans Y dans la plage de 1,5 à 4,4 % en poids et des éléments résiduels pour moins de 1 % en poids.

11. Formulation selon la revendication 5, **caractérisée en ce que** la formulation est un alliage de magnésium et contient du Y dans la plage de 3,7 à 5,5 % en poids, du Nd dans la plage de 1,8 à 2,7 % en poids et du Zr dans la plage de 0,2 à 1,2 % en poids.

12. Formulation selon la revendication 11, **caractérisée en ce que** l'alliage de magnésium est du WE43 (W25/EP5M).

13. Formulation selon l'une quelconque des revendications précédentes, **caractérisée en ce que** la formulation contient du Y et est adaptée de telle sorte qu'une concentration d'yttrium dans la zone des cellules musculaires lisses humaines à traiter se situe entre 200 µM et 2 mM, en particulier entre 800 µM et 1 mM.

14. Formulation selon l'une quelconque des revendications précédentes, **caractérisée en ce que** la formulation contient du Nd et est adaptée de telle sorte qu'une concentration de néodyme dans la zone des cellules musculaires lisses à traiter se situe entre 600 µM et 2 mM, en particulier entre 800 µM et 1 mM.

15. Formulation selon l'une quelconque des revendications précédentes, **caractérisée en ce que** la formulation contient du Zr et est adaptée de telle sorte qu'une concentration de zirconium dans la zone des cellules musculaires lisses à traiter se situe entre 200 µM et 2 mM, en particulier entre 200 µM et 1 mM.

16. Formulation selon l'une quelconque des revendications 1 à 12, **caractérisée en ce que** la formulation contient du Y, du Nd, et du Zr et est adapté de telle sorte qu'on ait une concentration d'yttrium entre 350 et 550 µM, une concentration de néodyme entre 100 et 200 µM et une concentration de zirconium entre 10 et 30 µM dans la zone des cellules musculaires lisses à traiter.

17. Implant avec un revêtement ou un composant à base d'une formulation selon l'une quelconque des revendications 4 à 16.

18. Implant selon la revendication 17, **caractérisé en ce que** l'implant est une structure de soutien endovasculaire, en particulier un stent.

19. Implant selon la revendication 18, **caractérisé en ce qu'**on a environ 5 à 30 µg d'yttrium, en particulier 10 à 20 µg d'yttrium, par rapport à une longueur de stent de 1 mm.

20. Implant selon la revendication 18, **caractérisé en ce qu'**on a environ 2 à 20 µg de néodyme, en particulier 3 à 10 µg de néodyme, par rapport à une longueur de stent de 1 mm.

21. Implant selon la revendication 18, **caractérisé en ce qu'**on a environ 0,05 à 10 µg de zirconium, en particulier 0,5 à 6 µg de zirconium, par rapport à une longueur de stent de 1 mm.
